# EUROPÄISCHE PATENTSCHRIFT

(11) **EP 1 421 968 B1**
(45) Veröffentlichungstag und Bekanntmachung des Hinweises auf die Patenterteilung: **14.10.2009**
(21) Anmeldenummer: 03025276.1
(22) Anmeldetag: 06.11.2003
(51) Int. Cl.: A61M 25/02

(54) **Vorrichtung zur Fixierung von Katheter und Filter**
Device for fixation of a catheter and a filter
Moyen de fixation d'un catheter et d'un filtre

(30) Priorität: 19.11.2002 DE 20217920 U
(43) Veröffentlichungstag der Anmeldung: 26.05.2004
(73) Patentinhaber: Pajunk GmbH & Co. KG Besitzverwaltung, 78187 Geisingen (DE)
(72) Erfinder: Pajunk, Heinrich, 78187 Geisingen (DE); Pajunk, Horst, 78187 Geisingen (DE)
(74) Vertreter: Mussgnug, Bernd

(56) Entgegenhaltungen:
- EP-A- 0 972 537
- FR-A- 2 351 348
- US-A- 4 561 857
- US-B1- 6 428 514

## Beschreibung

Die Erfindung betrifft eine Vorrichtung zur Fixierung von Katheter und Filter.

Wird einem Patienten ein Katheter, beispielsweise zum Applizieren eines Anästhetikums oder Medikaments, gelegt, besteht das Problem, wie Katheter und zugehöriger Filter am Patienten befestigt werden können. Bei Bewegung des Patienten darf der Katheter nicht versehentlich herausgezogen werden. Die Befestigung des Katheters und des Filters durch schlichtes Klebeband am Körper des Patienten kann in einer ungünstigen Lage bewirken, dass die Zufuhr des Anästhetikums abgedrückt wird. Zusätzlich ist bei dieser starren Fixierung die Gefahr, dass bei Bewegung des Patienten der Katheter herausgezogen wird, besonders groß.

Die DE 297 20 182 U1 beschreibt ein aufgeschäumtes Pflaster, welches auf die Haut des Patienten geklebt werden kann, mit einem senkrecht stehenden Stift, auf welchem der Filter beliebig drehbar anbringbar ist. Der Filter, der so über das Pflaster lokal an der Körperoberfläche des Patienten fixiert und über den Katheter mit dem Patienten verbunden ist, kann den Bewegungen des Patienten und des Katheters durch Drehung folgen. Allerdings muss auch hier der Katheter wiederum durch Klebeband am Patienten befestigt werden.

Aus der DE 1 954 956 ist eine Vorrichtung zum Befestigen eines Katheters bekannt, bei welcher der Katheter in einem Kanal eines Blocks eingeklemmt wird, wobei der Kanal einen Durchmesser aufweist, der dem Außendurchmesser des Katheters entspricht. Die Wirkung dieser Vorrichtung zum Befestigen eines Katheters entspricht im Wesentlichen der eines Klebebands, da eine bestimmte Stelle des Katheters definiert am Körper des Patienten fixiert wird.

Als weiterer Stand der Technik werden die gattungsgemäße US 4,561,857 und die EP 0 972 537 A2 genannt.

Die Aufgabe der Erfindung besteht darin, eine Vorrichtung bereitzustellen, welche Katheter und Filter zuverlässig am Patienten fixiert, ohne die Bewegungsfreiheit des Patienten zu sehr einzuschränken.

Die Aufgabe wird gelöst durch eine Vorrichtung zur Fixierung eines Filters und eines Katheters gemäß dem Patentanspruch 1.

Vorteilhafte Ausgestaltungen und Weiterbildungen der Erfindung sind in den Unteransprüchen angegeben.

Die erfindungsgemäße Vorrichtung zur Fixierung eines Filters und eines Katheters weist ein Klebepflaster, ein Befestigungselement zur Fixierung des Katheters und ein Trägerelement zur Fixierung des Filters auf, wobei entweder das Befestigungselement oder das Trägerelement auf dem Klebepflaster angebrachte ist, und wobei das Befestigungselement ein erstes Kupplungselement aufweist, welches mit einem an dem Trägerelement angebrachten zweiten Kupplungselement lösbar verbindbar ist. Somit ist es möglich, mit einer Vorrichtung gleichzeitig den Katheter und den Filter am Patienten zu fixieren.

Vorzugsweise weist das Befestigungselement Öffnungen auf, deren Achsen etwa parallel zur Ebene des Befestigungselements verlaufen. Diese Öffnungen nehmen den Katheter auf, wodurch er am Körper des Patienten fixiert wird.

In einer vorteilhaften Weiterbildung der Erfindung sind die Öffnungen derart ausgebildet, dass der Durchmesser der Öffnungen etwas größer ist als der Durchmesser des Katheters. Der Katheter ist somit durch das Befestigungselement positioniert, kann in den Öffnungen des Befestigungselements jedoch frei beweglich hin- und hergeschoben werden. Somit ist der Katheter des Patienten fixiert, jedoch soweit axial beweglich, so dass er jeder Bewegung des Patienten folgen kann und daher keine Gefahr besteht, dass der Katheter durch eine Bewegung des Patienten herausgezogen werden kann.

Vorzugsweise weist das Befestigungselement Spalte auf, welche an der Oberfläche des Befestigungselements offen sind und sich an ihrem Grund zu den Öffnungen erweitern. Statt den Katheter durch die Öffnungen zu fädeln und ihn erst anschließend in den Patienten einzuführen oder mit dem Filter zu verbinden, ist es dadurch möglich, den Katheter nachdem er dem Patienten gelegt und mit dem Filter verbunden wurde, durch die Spalte in die Öffnungen des Befestigungselements einzudrücken.

Vorteilhafterweise ist die Breite der Spalte geringfügig kleiner als der Durchmesser des Katheters. Auf diese Weise rastet der Katheter in der Öffnung ein und ist somit an der Scheibe fixiert, kann jedoch weiterhin frei axial beweglich in den Öffnungen des Befestigungselements hin- und hergeschoben werden.

Bei einer vorteilhaften Weiterbildung der Erfindung sind die Öffnungen in auf der Oberfläche des Befestigungselements angeordneten Stegen parallel zur Ebene des Befestigungselements eingelassen. Die Öffnungen sind somit besonders leicht zugänglich. Vorzugsweise sind die Stege in Form eines rechtwinkligen Kreuzes angeordnet.

Der Katheter kann als Schlaufe in zwei Öffnungen der Stege eingelegt werden. Bei Bewegungen des Patienten verschiebt sich der patientenferne Teil des Katheters in der Öffnung, wodurch sich die Schlaufe vergrößert bzw. verkleinert. Der patientennahe Teil des Katheters wird jedoch in dem Befestigungselement festliegend gehalten, so dass der Katheter nicht herausgezogen werden kann.

Vorzugsweise ist eines der beiden Kupplungselemente als Stift ausgebildet, während das andere der beiden Kupplungsteile als Öffnung ausgebildet ist, in welche der Stift einsetzbar ist. Vorzugsweise sind dabei der Stift und die Öffnung als Rastelemente ausgebildet, was eine besonders einfache Verbindung des Befestigungselements und des Trägerelements ermöglicht.

Vorteilhafterweise weisen der Stift und die Öffnung die Form eines einander entsprechenden Mehrkants, beispielsweise eines regelmäßigen Sechs- oder Achtecks auf. Der Filter kann somit in mehreren Positionen gegenüber dem Befestigungselement fixiert werden, abhängig davon, wie der Katheter relativ zum Patienten verläuft.

Vorzugsweise ist der Filter auf dem Trägerelement lösbar befestigt. Somit ist ein besonders einfaches Auswechseln des Filters möglich, während die Vorrichtung zur Fixierung von Katheter und Filter am Körper des Patienten verbleibt.

Vorteilhafterweise ist der Filter auf dem Trägerelement über eine Klemm- oder Rastfunktion befestigbar.

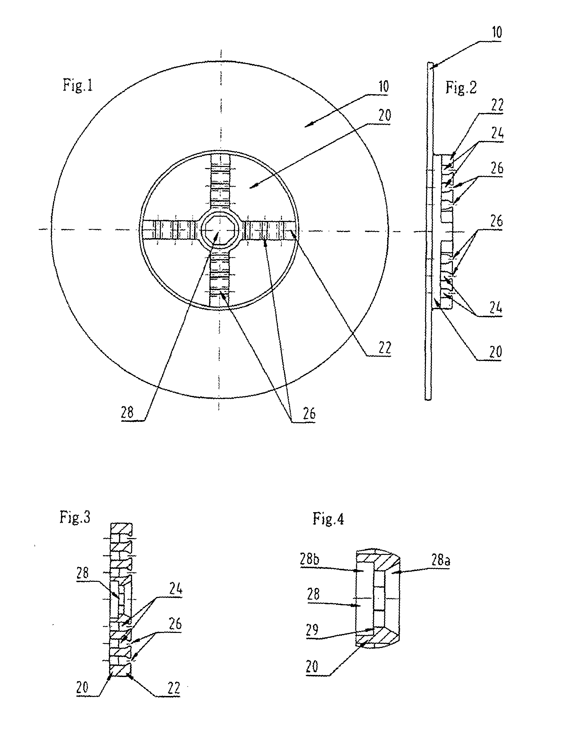

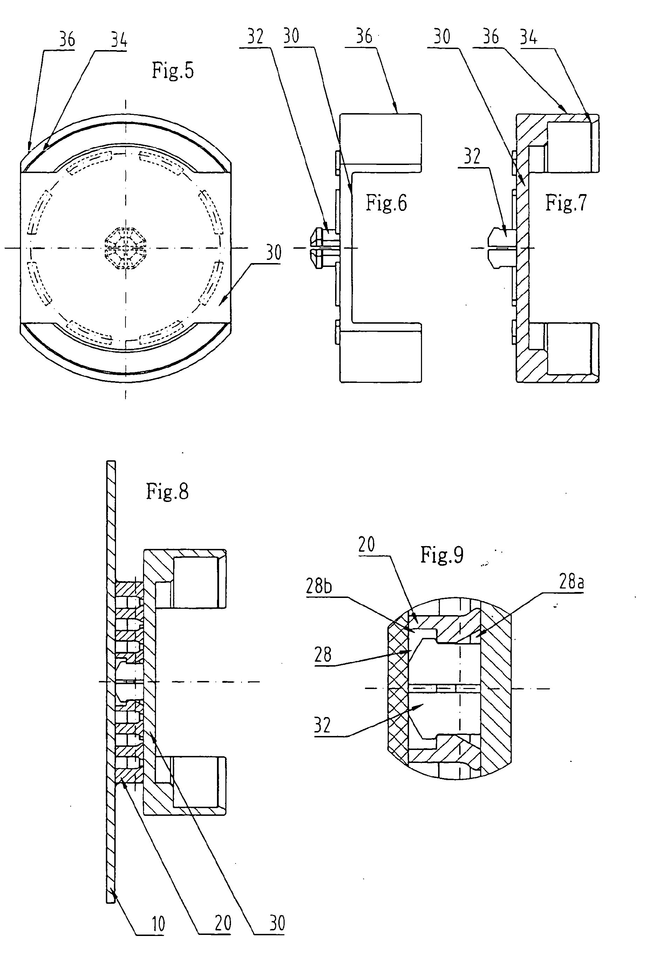

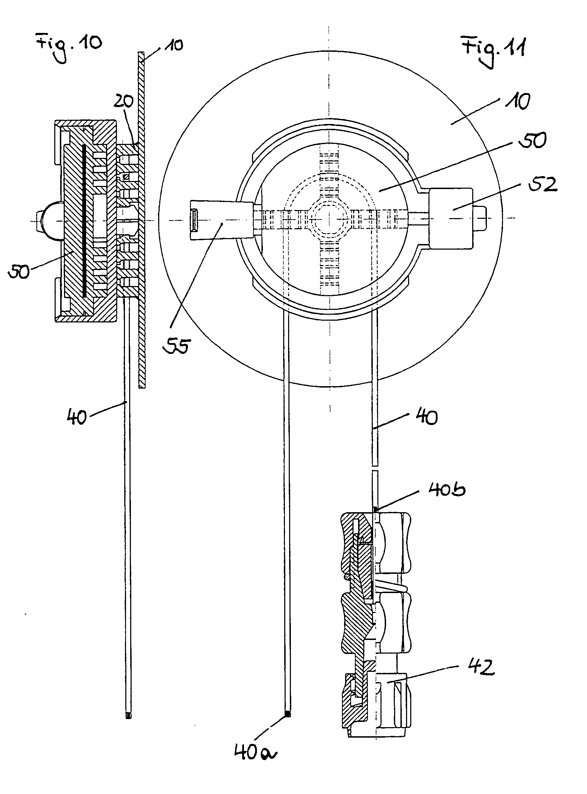

Ein Ausführungsbeispiel der Erfindung wird nachfolgend anhand der Zeichnung näher erläutert. Es zeigen:
Figur 1
   eine Draufsicht auf ein Pflaster mit einem Befestigungselement,
Figur 2
   eine Seitenansicht des Pflasters mit dem Befestigungselement,
Figur 3
   einen Axialschnitt des Befestigungselements,
Figur 4
   eine Ausschnittsvergrößerung aus Figur 3,
Figur 5
   eine Draufsicht auf ein Trägerelement,
Figur 6
   eine Seitenansicht des Trägerelements,
Figur 7
   einen Axialschnitt des Trägerelements,
Figur 8
   einen Axialschnitt durch ein Ausführungsbeispiel der Erfindung bestehend aus Pflaster, Befestigungselement und Trägerelement,
Figur 9
   eine Ausschnittsvergrößerung aus Figur 8,
Figur 10
   einen Axialschnitt durch ein Ausführungsbeispiel der Erfindung bestehend aus Pflaster, Befestigungselement und Trägerelement mit eingesetztem Filter und Katheter und
Figur 11
   eine Draufsicht auf das Ausführungsbeispiel aus Figur 10.

Figur 1 zeigt ein Ausführungsbeispiel der erfindungsgemäßen Vorrichtung zur Fixierung von Katheter und Filter bestehend aus einem Klebepflaster 10 und einem z. B. mit Klebstoff mittig darauf befestigten Befestigungselement 20. Das Klebepflaster 10 weist auf der Rückseite eine selbstklebende Schicht auf, mit welcher das Klebepflaster 10 am Körper eines Patienten befestigt werden kann.

Das Befestigungselement 20 besteht aus einer runden Scheibe aus Kunststoff, auf welcher zwei senkrecht zueinander und radial verlaufende Stege 22 angeformt sind. Die Stege 22 weisen parallel zur Ebene des Befestigungselements 20 und in tangentialer Richtung durchgehende Öffnungen 24 auf. Jeder radialen Steg 22 weist dabei drei Öffnungen 24 in gleichen radialen Abständen auf. Der Durchmesser der Öffnungen 24 ist etwas größer als der Durchmesser des zu befestigenden Katheters, um die freie axiale Beweglichkeit des Katheters in der Öffnung 24 zu garantieren.

Von der Oberseite der Stege 22 führt jeweils ein schmaler Spalt 26 in die Öffnung 24, über den der Katheter in die Öffnung 24 hineingedrückt werden kann (vgl. Figur 2). Der Durchmesser des Katheters ist dabei geringfügig größer als die Breite der Spalte 26, so dass sich der Katheter nach Einbringen in die Öffnungen 24 nicht ohne Kraftaufwendung wieder aus den Öffnungen 24 entfernen lassen kann. Der Katheter ist somit über das Befestigungselement 20 fixiert, kann jedoch entlang der Achse der Öffnungen 24 hin- und herbewegt werden.

Das Befestigungselement 20 weist mittig im Kreuzungspunkt der beiden Stege 22 eine axiale Öffnung 28 auf, in welche ein Trägerelement 30 über einen am Trägerelement 30 axial angebrachten Stift 32 eingesetzt werden kann. Die axiale Öffnung 28 weist ausgehend von der Oberfläche der Stege 22 einen konisch zulaufenden Abschnitt 28a auf, welcher über einen in der axialen Öffnung 28 umlaufenden Innenbund 29 in einen zylindrischen Abschnitt 28b übergeht (vgl. Figuren 3 und 4). Der Stift 32 ist ausgehend von der Unterseite des Trägerelements 30 zunächst zylindrisch ausgebildet, woran sich ein federnder Abschnitt mit geringfügig größerem Durchmesser und einem kreuzschlitzartigen Einschnitt anschließt (siehe Figuren 6 und 7). Wird das Trägerelement 30 auf das Befestigungselement 20 aufgesetzt, rastet der federnde Abschnitt mit geringfügig größerem Durchmesser des Stifts 32 hinter dem Innenbund 29 ein (vgl. Figuren 8 und 9). Das Trägerelement 30 wird über diese Kupplungsverbindung sicher und lösbar am Befestigungselement 20 gehalten.

Der Querschnitt der axialen Öffnung 28 und des Stifts 32 sind unrund ausgebildet und weisen z. B. die Form eines Achtecks auf. Das Trägerelement 30 kann somit in acht verschiedene Winkelpositionen relativ zum Befestigungselement 20 eingerastet werden, wobei es in jeder Position verdrehsicher gehalten wird.

Das Trägerelement 30 besteht aus einer runden Scheibe aus Kunststoff, deren Umfang durch zwei parallel verlaufende Sekanten abgeflacht ist (s. Figur 5). An den beiden verbleibenden Umfangsabschnitten ist senkrecht zur Ebene des Trägerelements 30 ein Rand 36 angeformt, welcher an seiner oberen Kante eine nach innen vorspringende umlaufende Feder 34 aufweist, die in eine entsprechende Nut eines nicht dargestellten einzusetzenden Filters einrastet.

Die Figuren 10 und 11 zeigen die Verwendung der erfindungsgemäßen Vorrichtung zur Fixierung von einem Katheter 40 und einem Filter 50. Um den Katheter 40 und den Filter 50 mittels der erfindungsgemäßen Vorrichtung an einem Patienten zu befestigen, wird zunächst das Klebepflaster 10 mittels der Klebeschicht an der Unterseite des Klebepflasters 10 auf die Haut des Patienten geklebt. Das eine Ende 40a des Katheters 40 wird dem Patienten gelegt. Anschließend wird der Katheter 40 in wenigstens eine, vorzugsweise unter Bildung einer Schlaufe in mindestens zwei der Öffnungen 24 des Befestigungselements 20 eingedrückt. Das proximale Ende 40b des Katheters 40 wird mit einem Anschlusselement 42 an ein entsprechendes am Filter 50 angeordnetes Anschlusselement 52 angeschlossen. Daraufhin wird das Trägerelement 30 mit dem Stift 32 in die axiale Öffnung 28 des Befestigungselements 20 eingesetzt und der Filter 50 auf das Trägerelement 30 geklemmt. Durch einen am Filter 50 angeordneten Anschluss 55 kann nun ein Anästhetikum oder ein Medikament appliziert werden.

Es ist ohne weiteres ersichtlich, dass auch das Trägerelement auf dem Klebepflaster angebracht sein kann. Auf das Trägerelement mit eingeklemmtem Filter wird dann mittels einer Kupplungseinheit das Befestigungselement aufgerastet, an welchem der Katheter fixierbar ist.

### Bezugszeichenliste

- 10: Klebepflaster

- 20: Befestigungselement
- 22: Steg
- 24: Öffnung
- 26: Spalt
- 28: Öffnung
- 28a: konischer Abschnitt
- 28b: zylindrischer Abschnitt
- 29: Innenbund

- 30: Trägerelement
- 32: Stift
- 34: Feder
- 36: Rand

- 40: Katheter
- 40a: Ende des Katheters
- 40b: Ende des Katheters
- 42: Anschlusselement

- 50: Filter
- 52: Anschlusselement
- 55: Anschluss

## Patentansprüche

1. Vorrichtung zur Fixierung eines Filters (50) und eines Katheters (40) mit einem Befestigungselement (20) zur Fixierung des Katheters (40) und ein Trägerelement (30) zur Fixierung des Filters (50),
**dadurch gekennzeichnet, dass**
entweder das Befestigungselement (20) oder das Trägerelement (30) auf einem Klebepflaster (10) aufgebracht ist, und dass das Befestigungselement (20) ein erstes Kupplungselement aufweist, welches mit einem an dem Trägerelement (30) angebrachten zweiten Kupplungselement lösbar verbindbar ist.

2. Vorrichtung nach Anspruch 1,
**dadurch gekennzeichnet, dass** das Befestigungselement (20) Öffnungen (24) aufweist, deren Achsen etwa parallel zur Ebene des Befestigungselements (20) verlaufen.

3. Vorrichtung nach Anspruch 2,
**dadurch gekennzeichnet, dass** der Durchmesser der Öffnungen (24) größer ist als der Durchmesser des Katheters (40).

4. Vorrichtung nach einem der Ansprüche 2 oder 3,
**dadurch gekennzeichnet, dass** das Befestigungselement (20) Spalte (26) aufweist, welche von der Oberfläche des Befestigungselements (20) in die Öffnungen (24) führen.

5. Vorrichtung nach Anspruch 4,
**dadurch gekennzeichnet, dass** die Breite der Spalte (26) geringfügig kleiner ist als der Durchmesser des Katheters (40).

6. Vorrichtung nach einem der Ansprüche 2 bis 5,
**dadurch gekennzeichnet, dass** die Öffnungen (24) in auf der Oberfläche des Befestigungselements (20) angeordneten Stegen (22) parallel zur Ebene des Befestigungselements (20) und senkrecht zu den Stegen (22) ausgebildet sind.

7. Vorrichtung nach Anspruch 6,
**dadurch gekennzeichnet, dass** die Stege (22) kreuzförmig angeordnet sind.

8. Vorrichtung nach einem der vorhergehenden Ansprüche,
**dadurch gekennzeichnet, dass** eines der beiden Kupplungselemente als Stift (32) ausgebildet ist, während das andere der beiden Kupplungselemente als Öffnung (28) ausgebildet ist, in welche der Stift (32) einsetzbar ist.

9. Vorrichtung nach Anspruch 8,
**dadurch gekennzeichnet, dass** der Stift (32) und die Öffnung (28) als Rastelemente ausgebildet sind.

10. Vorrichtung nach Anspruch 8 oder 9,
**dadurch gekennzeichnet, dass** der Stift (32) und die Öffnung (28) die Querschnittsform eines einander entsprechenden Mehrkants aufweisen.

11. Vorrichtung nach einem der vorhergehenden Ansprüche,
**dadurch gekennzeichnet, dass** der Filter (50) auf dem Trägerelement (30) lösbar befestigt ist.

12. Vorrichtung nach Anspruch 11,
**dadurch gekennzeichnet, dass** der Filter (50) auf dem Trägerelement (30) über eine Klemm- oder Rastfunktion befestigbar ist.

## Claims

1. Device for fixing a filter (50) and a catheter (40), comprising an attachment element (20) for fixing the catheter (40) and a carrier element (30) for fixing the filter (50), **characterised in that** either the attachment element (20) or the carrier element (30) is applied to an adhesive plaster (10), and **in that** the attachment element (20) comprises a first coupling element which can be releaseably connected to a second coupling element fitted on the carrier element (30).

2. Device according to claim 1, **characterised in that** the attachment element (20) comprises openings (24), the axes of which run approximately parallel to the plane of the attachment element (20).

3. Device according to claim 2, **characterised in that** the diameter of the openings (24) is larger than the diameter of the catheter (40).

4. Device according to one of claims 2 or 3, **characterised in that** the attachment element (20) comprises gaps (26) which lead from the surface of the attachment element (20) into the openings (24).

5. Device according to claim 4, **characterised in that** the width of the gaps (26) is slightly smaller than the diameter of the catheter (40).

6. Device according to one of claims 2 to 5, **characterised in that** the openings (24) are formed in webs (22) arranged on the surface of the attachment element (20), said openings being formed parallel to the plane of the attachment element (20) and perpendicular to the webs (22).

7. Device according to claim 6, **characterised in that** the webs (22) are arranged in the shape of a cross.

8. Device according to one of the preceding claims, **characterised in that** one of the two coupling elements is designed as a pin (32), while the other of the two coupling elements is designed as an opening (28), into which the pin (32) can be inserted.

9. Device according to claim 8, **characterised in that** the pin (32) and the opening (28) are designed as latching elements.

10. Device according to claim 8 or 9, **characterised in that** the pin (32) and the opening (28) have the cross-sectional shape of corresponding polygons.

11. Device according to one of the preceding claims, **characterised in that** the filter (50) is releaseably attached to the carrier element (30).

12. Device according to claim 11, **characterised in that** the filter (50) can be attached to the carrier element (30) via a clamping or latching function.

## Revendications

1. Dispositif de fixation d'un filtre (50) et d'un cathéter (40) avec un élément de fixation (20) pour fixer le cathéter (40) et un élément de support (30) pour fixer le filtre (50),
**caractérisé en ce que**
soit, l'élément de fixation (20) soit, l'élément de support (30) sont appliqués sur une bande adhésive (10) et **en ce que** l'élément de fixation (20) comporte un premier élément de couplage qui peut être relié de manière amovible à un second élément de couplage porté par l'élément de support (30).

2. Dispositif selon la revendication 1,
**caractérisé en ce que**
l'élément de fixation (20) comporte des ouvertures (24) dont les axes sont sensiblement parallèles au plan de l'élément de fixation (20).

3. Dispositif selon la revendication 2,
**caractérisé en ce que**
le diamètre des ouvertures (24) est supérieur au diamètre du cathéter (40).

4. Dispositif selon la revendication 2 ou 3,
**caractérisé en ce que**
l'élément de fixation (20) comporte des fentes (26) conduisant de la surface de l'élément de fixation (20) dans les ouvertures (24).

5. Dispositif selon la revendication 4,
**caractérisé en ce que**
la largeur de la fente (26) est légèrement inférieure au diamètre du cathéter (40).

6. Dispositif selon les revendications 2 à 5,
**caractérisé en ce que**
les ouvertures (24) sont réalisées dans les entretoises (22) prévues à la surface de l'élément de fixation (20), parallèles au plan de l'élément de fixation (20) et perpendiculaires aux entretoises (22).

7. Dispositif selon la revendication 6,
**caractérisé en ce que**
les entretoises (22) sont cruciformes.

8. Dispositif selon les revendications précédentes,
**caractérisé en ce que**
l'un des deux éléments de couplage est réalisé en forme de broche (32) alors que l'autre des deux éléments de couplage est réalisé sous la forme d'une ouverture (28) recevant la broche (32).

9. Dispositif selon la revendication 8,
**caractérisé en ce que**
la broche (32) et couverture (28) sont réalisées sous la forme d'éléments d'accrochage.

10. Dispositif selon la revendication 8 ou 9,
**caractérisé en ce que**
la broche (32) et couverture (28) ont en section la forme de polygones qui se correspondent.

11. Dispositif selon les revendications précédentes,
**caractérisé en ce que**
le filtre (50) est fixé de manière amovible à l'élément de support (30).

12. Dispositif selon la revendication 11,
**caractérisé en ce que**
le filtre (50) est fixé à l'élément de support (30) par une fonction de serrage ou d'accrochage.
